# EUROPÄISCHE PATENTSCHRIFT

(11) **EP 0 545 179 B1**
(45) Veröffentlichungstag und Bekanntmachung des Hinweises auf die Patenterteilung: **06.12.1995**
(21) Anmeldenummer: 92119848.7
(22) Anmeldetag: 21.11.1992
(51) Int. Cl.: C07C 11/02, C07C 5/27

(54) **Verfahren zur Skelettisomerisierung von n-Alkenen**
Process for the skeletal isomerisation of n-alkenes
Procédé pour l'isomérisation du squelette de n-alcènes

(30) Priorität: 30.11.1991 DE 4139552
(43) Veröffentlichungstag der Anmeldung: 09.06.1993
(73) Patentinhaber: LEUNA-WERKE GMBH, D-06236 Leuna (DE); VEREINIGTE ALUMINIUM-WERKE AKTIENGESELLSCHAFT, D-53117 Bonn (DE); VEBA OEL AG, 45876 Gelsenkirchen (DE)
(72) Erfinder: Timm, Dieter, Dr., O-4090 Halle (DE); Öhlmann, Gerhard, Prof. Dr., O-1020 Berlin (DE); Fricke, Rolf, Dr., O-1055 Berlin (DE); Roost, Udo, O-1055 Berlin (DE); Zubowa, Heide-Lore, Dr., O-1153 Berlin (DE); Becker, Karl, Dr., O-4803 Bad Kösen (DE); Striegler, Helmut, Dr., O-4090 Halle (DE)
(74) Vertreter: Schinke, Herbert, Dr. Dr., Patentanwaltskanzlei

(56) Entgegenhaltungen:
- EP-A- 0 103 117
- EP-A- 0 131 946
- EP-A- 0 132 708
- EP-A- 0 158 348
- EP-A- 0 158 975
- EP-A- 0 161 489
- EP-A- 0 161 490
- EP-A- 0 161 491

## Beschreibung

Die Erfindung betrifft ein Verfahren zur Skelettisomerisierung von n-Alkenen zu Isoalkenen bzw. zur Anreicherung von Isoalkenen in n-alkenhaltigen Kohlenwasserstoffgemischen.

Es ist bereits bekannt, n-Alkene katalytisch zu Isoalkenen zu isomerisieren. Dabei werden halogenenthaltende sowie halogenfreie Katalysatoren zur Isomerisierung eingesetzt. Mit halogenenthaltenden Katalysatoren wurden in einigen Fällen Katalysatorlaufzeiten von 100 h erreicht (DE 3040698, DE 3137383). Diese Katalysatoren besitzen jedoch den Nachteil, daß ihr Halogenanteil stetig mit dem Isomerisierungsprodukt ausgetragen wird, insbesondere wenn es wasserhaltig ist, wodurch sie ständig an Wirksamkeit verlieren. Zum Erhalt der katalytischen Aktivität ist es daher erforderlich, dem Katalysator fortlaufend neues Halogen zuzuführen. Ein weiterer Nachteil bei dem Einsatz solcher Katalysatoren besteht darin, daß halogenbeständige Werkstoffe für die Apparate verwendet werden müssen.

Es ist weiterhin bekannt, in halogenfreier Arbeitsweise oxidische Katalysatoren auf Basis Aluminiumoxid, Siliziumdioxid, ggf. mit Zusätzen weiterer Oxide (US 2216285, SU 551315) von Metallen oder ihren Verbindungen (DE 2059619, ER 66485) oder oberflächenbehandelt mit siliziumorganischen Verbindungen (DE 3340958, ES 440497, US 4038337, SU 137727) einzusetzen.

Es ist außerdem bekannt, phosphathaltige (DE 971849, US 2281804, US 2554202, US 3448164, GB 602202, GB 635508) bzw. phosphorsäurehaltige (US 2220693, FR 823545) sowie sulfatenthaltende (F. K. Serebryakova, Compt. rend. acad. sci (UdSSR) IV, 359 (1936); J. C. Luy, Reakt. Kin. Cat. Lett., Vol. 36, No. 2 273-279 (1988)) Katalysatoren zu verwenden. Alle diese Katalysatoren haben jedoch den Nachteil, daß mit ihnen nur Katalysatorlaufzeiten in einer Fahrperiode von unter 70 h erreicht werden und/oder eine erhebliche Nebenproduktbildung eintritt.

Es ist fernerhin beschrieben, daß mikroporöse Alumophosphate, die neben Aluminium und Phosphor noch Eisen (EP 0131946), Magnesium, Mangan, Kobalt, Zink (EP 0132708), Eisen, Silizium (EP 0161491), Mangan, Silizium (EP 0161490), Kobalt, Silizium (EP 0161489), Magnesium, Silizium (EP 0158348), Zink, Silizium (EP 0158975) oder Silizium (EP0103117) enthalten, für alle Kohlenwasserstoffumwandlungsverfahren, so auch zur Isomerisierung von Olefinen geeignet sind. Außer der allgemeinen Angabe über die Reaktionstemperatur (500 - 900 °F) fehlen jedoch weitere Angaben zur spezifischen Wirksamkeit für diese Reaktion. Ebenso fehlen Ausführungsbeispiele dazu. Eigene Versuche von Olefinisomerisierungen mit den gemäß EP 131946 und EP 161490 synthetisierten Katalysatoren ergaben, daß damit nur eine Isobutenselektivität von weniger als 60 % und eine Laufzeit der Katalysatoren von unter 50 Stunden erreichbar war.

Ziel der Erfindung ist es, die geschilderten Nachteile der bekannten Verfahren zu vermeiden. Es bestand somit die Aufgabe, ein Verfahren zur Skelettisomerisierung von n-Alkenen zu Isoalkenen bzw. zur Anreicherung von Isoalkenen in alkenhaltigen Kohlenwasserstoffgemischen zu entwickeln, bei dem Katalysatorlaufzeiten von über 100 h in einer Fahrperiode unter Vermeiden des Einsatzes halogenenthaltender Katalysatoren erreicht werden können.

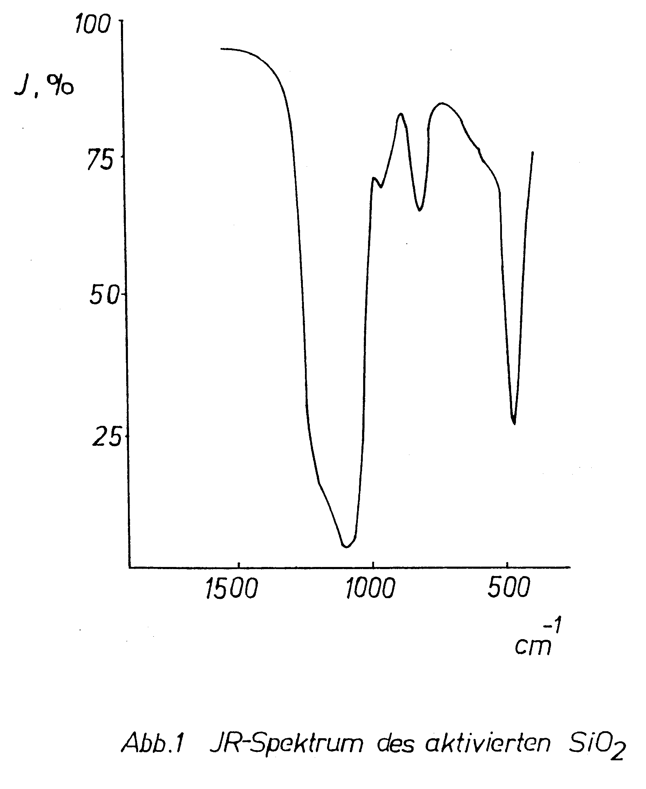

Diese Aufgabe wird durch ein Verfahren zur Skelettisomerisierung von n-Alkenen zu Isoalkenen bzw. zur Anreicherung von Isoalkenen in alkenhaltigen Kohlenwasserstoffgemischen bei Temperaturen von 593 bis 823 K und Drücken von 98 bis 2100 kPa, ggf. in Gegenwart von Inertgasen und/oder von Wasserstoff, an einem mikroporösen Alumophosphatkatalysator mit Molekularsiebstruktur gelöst, wobei erfindungsgemäß als Isomerisierungskatalysator ein Alumophosphat mit Poreneingangsöffnungen von 0,4 bis 0,6 nm Durchmesser verwendet wird, zu dessen Herstellung aktiviertes Siliziumdioxid eingesetzt wird, das ein IR-Spektrum gemäß Abb. 1 besitzt, und dieses Alumophosphat mit einem Bindemittel gemischt und in bekannter Weise verformt und anschließend bei Temperaturen von 623 bis 873 K aktiviert wird. Die erfindungsgemäßen Katalysatoren zeigen überraschenderweise eine deutlich höhere Olefinisomerisierungsaktivität und Katalysatorlaufzeit als die bisher bekannten Alumophosphate.

Als vorteilhaft haben sich für die Skelettisomerisierung von n-Alkenen zu Isoalkenen dabei Alumophosphate erwiesen, die Silizium und/oder zweiwertige Metalle im Gerüst und/oder Gitter enthalten und die im Röntgendiffraktogramm (Cu-K-alpha-Strahlung) durch Interferenzen in mindestens folgenden Netzebenenbereichen gekennzeichnet sind:
d (A) : 2,8 bis 3,0; 3,8 bis 4,1; 4,0 bis 4,5; 10 bis 12
Solche Alumophosphate kristallisieren aus gelartigen Reaktionsgemischen, die aus einer Aluminiumverbindung, einer Phosphorverbindung, einer Siliziumverbindung, Wasser, ggf. einer Verbindung von zweiwertigen Metallen und einer strukturdirigierenden Verbindung bestehen, bei Temperaturen zwischen 423 und 473 K unter autogenem Druck in z. B. mit Teflon ausgekleideten Stahlautoklaven. Als strukturdirigierende Verbindungen können sekundäre Amine oder quaternäre Ammoniumverbindungen verwendet werden. Die Siliziumquelle ist aktiviertes SiO₂, z. B. durch einen vorgeschalteten Mahlprozeß hergestellt, das vorwiegend sphärische Partikel mit einem Durchmesser von etwa 2 nm und das in Abbildung 1 wiedergegebene IR-Spektrum besitzt.

Als Phosphorverbindung wird zweckmäßigerweise Orthophosphorsäure, als Aluminiumquelle werden Aluminiumoxidhydratverbindungen, vorwiegend Pseudoböhmit oder Aluminiumhydroxid eingesetzt. Als Metallverbindungen werden vorwiegend Sulfate, Acetate und Chloride von Zink, Mangan, Magnesium, Eisen, Kobalt sowie deren Mischungen verwendet. Auch Kombinationen mit hydrieraktiven Komponenten in Anteilen von 0,01 bis 2 Masse-%, bezogen auf den Katalysator, insbesondere Palladium, sind geeignet. Für das Reaktionsgemisch geeignete molare Verhältnisse sind
SiO₂/Al₂O₃ - 0,08 bis 0,5
P₂O₅/Al₂O₃ - 0,8 bis 1,2
H₂O/Al₂O₃ - 30,0 bis 60,0
MeO/Al₂O₃ - 0,004 bis 0,025
Das molare Verhältnis der strukturdirigierenden Verbindung zum Al₂O₃-Anteil liegt im Bereich von 0,8 bis 1,2. Die aktivierte Katalysatorkomponente wird in bekannter Weise nach Mischen mit einem Bindemittel durch z. B. Extrusion, Vertropfen, Agglomerieren oder Verpressen verformt und anschließend bei 623 bis 873 K aktiviert. Als Bindemittel sind besonders Pseudoböhmit, Kieselsäure oder Schichtsilikate, insbesondere Magadiit, geeignet. Die genannten Katalysatoren sind zur Skelettisomerisierung von n-Alkenen zu Isoalkenen bzw. zur Anreicherung von Isoalkenen in n-alkenhaltigen Kohlenwasserstoffgemischen hervorragend geeignet.

Zur Skelettisomerisierung werden lineare, isomerisierbare Olefine, rein oder im Gemisch mit anderen Kohlenwasserstoffen, insbesondere Alkanen, getrocknet oder wasserhaltig, z. B. wassergesättigt, eingesetzt. Die olefinhaltigen Gemische können z. B. aus der Aufarbeitung von Pyrolyseprodukten stammen oder Restgasgemische aus Synthesen, z. B. aus der Synthese von Methyl-tert.-butylether, sein. Sie können mehrfach ungesättigte Verbindungen enthalten oder zu deren Beseitigung hydriert worden sein. Sie können auch aus der Hydrierung von Diolefinen, z. B. Butadien-1,3, zu Monoolefinen stammen. Die Olefinisomerisierung kann unter Zusatz von bis zu 95 Vol.-% Inerten, wie Stickstoff, Kohlendioxid sowie Wasserstoff oder solchen enthaltenden Gasen oder von Wasser durchgeführt werden. Bei angestrebtem weitgehendem Umsatz der n-Olefine zu Isoolefinen ist ein Produktkreislauf zweckmäßig.
Die Katalysatorbelastung liegt bei 1 bis 30 g/g Katalysator . h, bevorzugt bei 2 bis 10 g/g Katalysator . h, bezogen auf das Olefin. Die Reaktionstemperatur liegt bei 593 bis 823 K. Man kann bei hohem Gehalt des Einsatzproduktes an 1-Olefinen, beispielsweise einem hohen Gehalt an Buten-1, die Isomerisierung auch zweistufig durchführen und bei Temperaturen unter 573 K zunächst eine Doppelbindungsisomerisierung, z. B. die von Buten-1 in Buten-2, durchführen und das dabei erhaltene Produkt dann bei Temperaturen über 573 K am Skelett isomerisieren, z. B. zu Isobuten. Natürlich ist das in der 1. Stufe erhaltene, z. B. an Buten-2 reiche Gemisch auch für andere Synthesen einsetzbar, z. B. zur Aromatenalkylierung.

Der Reaktionsdruck ist nicht kritisch. Zur Isomerisierung erfolgt zweckmäßigerweise eine Anpassung an den Anlagendruck verbundener Apparaturen, z. B. der das Ausgangsgemisch liefernden Anlage oder der der Aufarbeitung. Bevorzugt wird im Druckbereich zwischen 98 und 2100 kPa gearbeitet. Die Zusammensetzung der Reaktionsprodukte wurde gaschromatographisch an mit Silbernitrat gesättigtem Benzylcyanid auf Porolith bestimmt.

Mit den beschriebenen Katalysatoren, die im Fest- und Wirbelbett einsetzbar sind, wird unter den genannten Reaktionsbedingungen eine hohe Selektivität an Isoolefin erreicht, wobei das Reaktionsprodukt Spalt- oder höhersiedende Komponenten nur in Anteilen von weniger als 3 bzw. 2 % enthält. Die Katalysatorlaufzeit in einer Fahrperiode liegt unter günstigen Reaktionsbedingungen bei über 300 h. Bei Nachlassen der Katalysatoraktivität läßt sich seine Leistungsfähigkeit in üblicher Weise durch Abbrennen des niedergeschlagenen Kohlenstoffs mit Sauerstoff oder sauerstoffhaltigen Gasen, ggf. unter Zusatz von Wasserdampf, bei 673 bis 973 K wieder herstellen.

### Beispiel 1

8,14 g Pseudoböhmit, 21,3 g Wasser und 1,75 g aktiviertes Siliziumdioxid (IR-Spektrum siehe Abbildung 1) wurden miteinander vermischt und zwei Stunden gerührt. Zu dem Gemisch wurden 11,5 g konzentrierte Orthophosphorsäure gegeben und weiter verrührt. Nach Erreichen der gewünschten Homogenität wurde die Suspension mit 11,0 g Wasser verdünnt und mit 5,1 g Di-n-Propylamin versetzt. Das Reaktionsgel wies folgende Zusammensetzung auf: 0,8 Di-n-Propylamin : 1,0 Al₂O₃ : 0,8 P₂O₅ : 0,5 SiO₅ : 33,4 H₂O (Mol-Teile). Die Alterung des Gels erfolgte bei 298 ± 2 K und die Kristallisation in einem Teflonbehälter in einem Stahlautoklaven (Volumen 90 ml) bei 473 ± 2 K in 24 h. Das Kristallisationsprodukt wurde von der Mutterlauge getrennt, mit destilliertem Wasser neutral gewaschen und bei 303 K getrocknet. Zur Beseitigung der strukturdirigierenden Verbindung wurde die Probe sieben Stunden bei 873 ± 2 K in Luft geglüht. Die Röntgenaufnahmen der Proben wiesen Reflexe, wie in der Tabelle angegeben, auf. Porendurchmesser: 0,55 nm.

Aus 4,0 g Aktivkomponente, 2,84 g Pseudoböhmit, 0,12 ml Salpetersäure (65,0 Masse-%ig, p.a.), 0,40 g Stärkekleister (9,0 Masse-% Stärke) und 3,7 g Wasser wurde eine Paste hergestellt und zu Strängen verformt. Die Stränge wurden in den Stufen 1 h bei 413 K, 1 h bei 513 K, 3 h bei 873 K getempert.

**Tabelle**

| Röntgenreflexe | |
|---|---|
| d | Intensität |
| 10,28 - 10,65 | sehr stark |
| 4,31 - 4,40 | schwach bis stark |
| 4,00 - 4,21 | schwach bis stark |
| 3,80 - 3,99 | sehr stark |
| 2,80 - 2,82 | schwach |

### Beispiel 2

Für die Synthese eines Mangansilicoalumophosphat-Molsiebes wurden 8,14 g Pseudoböhmit, 0,34 g MnSO₄ . 4 H₂O (p.a.), 0,29 g durch Mahlen aktiviertes SiO₂und 31,0 g Wasser vorgelegt und 1 h bei Raumtemperatur gerührt. Kurz vor Beendigung der Rührzeit wurden 11,3 g Orthophosphorsäure (85 Masse-%ig) hinzugefügt. Es wurde dann eine weitere Stunde gerührt. Danach erfolgte eine Verdünnung mit 30,0 g Wasser und die Zugabe von 7,26 g Di-n-Propylamin. Ein halbstündiges intensives Rühren war auf Grund einer plötzlich eintretenden Suspensionsverdichtung erforderlich. Das Gel der Zusammensetzung 1,2 Di-n-Propylamin : 1,0 Al₂O₃ : 0,82 P₂O₅ : 0,025 MnO : 0,08 SiO₂ : 60 H₂O (Mol-Teile) kristallisierte unter den in Beispiel 1 angegebenen Bedingungen. Das Kristallisationsprodukt wurde wie in Beispiel 1 nachbehandelt. Das Produkt hatte die in der Tabelle angegebenen Röntgenreflexe. 2,0 g templatfreie Substanz wurde mit 1,08 g Aerosil 200 (Fa. Degussa) und 5,0 g Wasser zu einer Paste vermischt und mit einer Strangpresse zu Strängen von 1 mm Durchmesser und einer mittleren Länge von 3 mm verarbeitet, die bei 393 K getrocknet wurden. Porendurchmesser: 0,54 nm.

### Beispiel 3:

Analog Beispiel 2 erfolgte die Herstellung eines Zinksilicoalumophosphat-Molsiebes. Bei der Gelzubereitung wurden entsprechend 0,07 g ZnSO₄ . 7 H₂O (p.a.) verwendet. Die Gelzusammensetzung war 1,2 Di-n-Propylamin : 1,0 Al₂O₃ : 0,82 P₂O₅ : 0,004 ZnO : 0,8 SiO₂ : 60 H₂O (Mol-Teile), Porendurchmesser: 0,54 nm.

### Beispiel 4

0,29 aktiviertes Siliziumdioxid, 0,35 g FeSO₄ . 7 H₂O (p.a. ) 16,59 g Orthophosphorsäure, 30,5 g Wasser wurden im Erlenmeyerkolben 1 mit einem Magnetrührer 15 Minuten gemischt. Parallel dazu wurden in einem zweiten Kolben 8,14 g Pseudoböhmit, 30,0 g Wasser und 7,26 g Di-n-Propylamin 15 Minuten verrührt. Der Inhalt des Kolbens 1 wurde in den Kolben 2 gegeben. Es wurde nochmals 30 Minuten gerührt. Nachfolgend wurde mit dem Gel der Zusammensetzung 1,2 Di-n-Propylamin : 1,0 Al₂O₃ : 1,2 P₂O₅ : 0,08 SiO₂ : 0,02 FeO : 60 H₂O (Mol-Teile) wie im Beispiel 2 verfahren. Der Porendurchmesser betrug 0,55 nm.

### Beispiel 5

Analog Beispiel 4 erfolgte die Herstellung eines Magnesiumsilicoalumophosphat-Molsiebes. Bei der Gelzubereitung wurden entsprechend 0,31 g MgSO₄ . 7 H₂O verwendet. Die Gelzusammensetzung war 1,2 Di-n-Propylamin : 1,0 Al₂O₃ : 1,2 P₂O₅ : 0,08 SiO₂ : 0,02 MgO : 60 H₂O (Mol-Teile), Porendurchmesser: 0,55 nm.

### Beispiel 6

In einen Reaktor von 430 mm Länge und einem Durchmesser von 25 mm wurden 50 ml eines Katalysators nach Beispiel 2 gegeben. Der Katalysator wurde bei 743 K und einem Gesamtdruck von 102 kPa mit 3 g/g Katalysator . h eines Restkohlenwasserstoffgemisches aus der Synthese von Methyl-tert.-butylether der Zusammensetzung (in Masse-%)

| | |
|---|---|
| Propan | 0,05 |
| Propen | 0,12 |
| Isobutan | 4,24 |
| n-Butan | 17,40 |
| Isobuten | 0,18 |
| Buten-1 | 48,62 |
| trans-Buten-2 | 12,21 |
| cis-Buten-2 | 17,10 |

belastet.

Nach 315 h Belastungszeit wurden ein Umsatz der n-Butene von 42,1 %, eine Isobutenselektivität von 93,3 % und eine Isobutenausbeute von 39,3 % erhalten. Nach 320 h wurde die Zuführung des Kohlenwasserstoffgemisches beendet und der Katalysator unter Strömung von 5 l/h Stickstoff auf 773 K hochgeheizt. Nach Erreichen dieser Temperatur wurde zur Regeneration des Katalysators 6 h mit Luft behandelt. Dann wurde die Reaktortemperatur unter 5 l/h Stickstoffströmung auf 743 K gesenkt. Nach Erreichen dieser Temperatur wurde die Stickstoffströmung abgestellt und der Katalysator unter den oben genannten Bedingungen erneut mit dem Kohlenwasserstoffgemisch belastet. Nach weiteren vier Regenerierungen wurden nach 2016 h ein Umsatz der n-Butene von 38,4 %, eine Isobutenselektivität von 87,1 % und eine Isobutenausbeute von 33,4 % erhalten.

### Beispiel 7

In einen Reaktor entsprechend Beispiel 6 wurden 50 ml eines Katalysators nach Beispiel 1 gegeben. Der Katalysator wurde bei 773 K und einem Gesamtdruck von 102 kPa mit 2 g/g Katalysator . h eines Restkohlenwasserstoffgemisches aus der Synthese von Methyl-tert.-butylether der Zusammensetzung wie im Beispiel 6, unter weiterer Einspeisung von 25 g/h Wasser, belastet. Nach 280 h wurden ein n-Butenumsatz von 39,5 %, eine Isobutenselektivität von 83,2 % und eine Isobutenausbeute von 32,9 % erhalten.

### Beispiel 8

In einen Reaktor entsprechend Beispiel 6 wurden 50 ml eines Katalysators nach Beispiel 2 gegeben. Der Katalysator wurde bei 823 K und einem Gesamtdruck von 2040 kPa mit 9 g/g Katalysator . h eines Kohlenwasserstoffgemisches der Zusammensetzung (in Masse-%)

| | |
|---|---|
| Propan | 0,09 |
| Propen | 0,19 |
| Isobutan | 7,16 |
| n-Butan | 12,76 |
| Isobuten | 0,25 |
| Buten-1 | 57,93 |
| trans-Buten-2 | 18,29 |
| cis-Buten-2 | 23,22 |
| C₅+-Kohlenwasserstoffe | 0,11 |

belastet. Nach 260 h wurden ein Umsatz der n-Butene von 35,7 %, eine Isobutenselektivität von 88,6 % und eine Isobutenausbeute von 31,6 % erhalten.

### Beispiel 9

In einen Reaktor entsprechend Beispiel 6 wurden 50 ml eines Katalysators nach Beispiel 2 gegeben. Der Katalysator wurde bei 673 K und einem Gesamtdruck von 102 kPa mit 2 g/g Katalysator . h eines Restkohlenwasserstoffgemisches aus der Synthese von Methyl-tert.-butylether der Zusammensetzung

| | |
|---|---|
| Propan | 1,09 |
| Propen | 0,74 |
| Isobutan | 12,81 |
| n-Butan | 8,25 |
| Isobuten | 0,28 |
| Buten-1 | 59,90 |
| trans-Buten-2 | 7,12 |
| cis-Buten-2 | 9,40 |
| C₅+-Kohlenwasserstoffe | 0,36 |
| Butadien | 0,05 |

unter weiterer Einspeisung von 20 l/Wasserstoff, belastet. Nach 275 h wurden ein Umsatz der n-Butene von 37,2 %, eine Isobutenselektivität von 91,1 % und eine Isobutenausbeute von 33,9 % erhalten.

### Beispiel 10

In einen Reaktor entsprechend Beispiel 6 wurden 50 ml eines Katalysators nach Beispiel 2 gegeben. Der Katalysator wurde bei 733 K und einem Gesamtdruck von 102 kPa mit 3 g/g Katalysator . h eines Butengemisches aus 98,2 % Buten-1, 0,7 % transButen-2 und 1,1 % cis-Buten-2 belastet. Nach 328 h wurden ein Umsatz der n-Butene von 41,6 %, eine Isobutenselektivität von 92,2 % und eine Isobutenausbeute von 38,4 % erhalten.

### Beispiel 11

In einen Reaktor entsprechend Beispiel 6 wurden 50 ml eines Katalysators nach Beispiel 4 gegeben. Der Katalysator wurde bei 713 K und einem Gesamtdruck von 150 kPa mit 7 g/g Katalysator . h eines Butengemisches entsprechend Beispiel 10, unter weiterer Einspeisung von 10 l/h Wasserstoff, belastet. Nach 310 h wurden ein Umsatz der n-Butene von 36,9 %, eine Isobutenausbeute von 31,9 % und eine Isobutenselektivität von 86,4 % erhalten.

### Beispiel 12

In einen Reaktor entsprechend Beispiel 6 wurden 50 ml eines Katalysators nach Beispiel 3 gegeben. Der Katalysator wurde bei 703 K und einem Gesamtdruck von 150 kPa mit 2 g/g Katalysator . h eines Butengemisches entsprechend Beispiel 10, unter weiterer Einspeisung von 50 g/h Wasser, belastet. Nach 160 h wurden ein Umsatz der n-Butene von 35,2 %, eine Isobutenselektivität von 91,2 % und eine Isobutenausbeute von 32,1 % erhalten.

### Beispiel 13

In einen Reaktor entsprechend Beispiel 6 wurden 50 ml eines Katalysators nach Beispiel 1, auf den durch Tränkung mit Palladiumnitrat noch 0,01 Masse-% Palladium aufgebracht wurden, gegeben. Der Katalysator wurde bei 733 K und einem Gesamtdruck von 102 kPa mit 3 g/g Katalysator . h eines Restkohlenwasserstoffgemisches aus der Synthese von Methyl-tert.-butylether der in Beispiel 6 genannten Zusammensetzung, unter weiterer Einspeisung von 30 l/h Wasserstoff, belastet. Nach 360 h wurden ein Umsatz der n-Butene von 39,8 %, eine Isobutenselektivität von 92,0 % und eine Isobutenausbeute von 36,6 % erhalten.

### Beispiel 14

In einen Reaktor entsprechend Beispiel 6 wurden 50 ml eines Katalysators nach Beispiel 2 gegeben. Der Katalysator wurde bei 503 K und einem Gesamtdruck von 150 kPa mit 3 g/g Katalysator . h eines Butengemisches aus 98,2 % Buten-1, 0,7 % transButen-2 und 1,1 % cis-Buten-2 belastet. Nach 240 h wurden ein Umsatz an n-Buten-1 von 78 ,3 %, eine Selektivität an cis-Buten-2 von 37,5 % und von trans-Buten-2 von 62,5 % erhalten. Das gesamte Reaktionsprodukt wurde über 50 ml des gleichen Katalysators in einen zweiten Reaktor bei einer Belastung von 3 g/g Katalysator . h mit Butengemisch bei einer Temperatur von 743 K und einen Gesamtdruck von 150 kPa geleitet. Nach 240 h wurden ein Umsatz der n-Butene von 40,1 % und eine Isobutenselektivität von 93,2 % und eine Isobutenausbeute von 37,4 % erhalten.

### Beispiel 15

In einen Reaktor entsprechend Beispiel 6 wurden 50 ml eines Katalysators nach Beispiel 5 gegeben. Der Katalysator wurde bei 663 K und einem Gesamtdruck von 150 kPa mit 3 g/g Katalysator . h mit einem Kohlenwasserstoffgemisch der Zusammensetzung (in Masse-%)

| | |
|---|---|
| C₃-Kohlenwasserstoffe | < 0,05 |
| Isobutan | 4,1 |
| n-Butan | 15,3 |
| Isobuten | 42,5 |
| Buten-1 | 22,7 |
| trans-Buten-2 | 8,8 |
| cis-Buten 2 | 6,1 |
| Butadien | 0,3 |
| C₅+-Kohlenwasserstoffe | < 0,1 |
| Wasser | < 0,05 |

belastet. Nach 335 h wurden ein Umsatz der n-Butene von 35,6 %, eine Isobutenselektivität von 92,3 % und eine Isobutenausbeute von 32,9 % erhalten.

### Beispiel 16

1,84 g eines Katalysators nach Beispiel 2 wurden in einen elektrisch beheizten Stahlreaktor von 10 mm Durchmesser und 200 mm Länge eingebracht und bei 743 K mit 3,6 l/h eines Gemisches von 14,5 Vol.-% Hexen-1 in Stickstoff belastet. Das Reaktionsprodukt wurde ausgefroren und gaschromatographisch analysiert. Es hatte folgende Zusammensetzung (in Masse-%)

| | |
|---|---|
| 3-Methylpent-1-en | 3,2 |
| 2.3-Dimethylbut-1-en | 5,6 |
| 4-Methylpent-2-en (cis+trans) | 4,6 |
| 2-Methylpent-1-en | 10,0 |
| n-Hexene (-1, -2, -3) | 23,0 |
| 2-Ethylbut-1-en | 5,4 |
| 2-Methylpent-2-en | 16,5 |
| 2-Methylpent-2-en (cis) | 9,2 |
| 3-Methylpent-2-en (trans) | 13,8 |
| Methylcyclopentan | 3,2 |
| 3,3-Dimethylbut-2-en | 5,5 |

## Patentansprüche

1. Verfahren zur Skelettisomerisierung von n-Alkenen zu Isoalkenen, bzw. zur Anreicherung von Isoalkenen in alkenhaltigen Kohlenwasserstoffgemischen, bei Temperaturen von 593 bis 823 K und Drücken von 98 bis 2100 kPa, ggf. in Gegenwart von Inertgasen und/oder Wasserstoff, an einem mikroporösen Alumophosphatkatalysator mit Molekularsiebstruktur, gekennzeichnet dadurch, daß als Isomerisierungskatalysator ein mikroporöses Alumophosphat mit Poreneingangsöffnungen von 0,4 bis 0,6 nm Durchmesser verwendet wird, zu dessen Herstellung aktiviertes Siliziumdioxid eingesetzt wird, das ein IR-Spektrum gemäß Abb. 1 besitzt, und dieses Alumophosphat mit einem Bindemittel gemischt und in bekannter Weise verformt und anschließend bei Temperaturen von 623 bis 873 K aktiviert wird.

2. Verfahren gemäß Anspruch 1, gekennzeichnet dadurch, daß Alumophosphate verwendet werden, die Silizium und/oder zweiwertige Metalle im Gerüst und/oder Gitter enthalten und ein Röntgendiffraktogramm (Cu-K-alpha-Strahlung), das durch Interferenzen in mindestens folgenden Netzebenenbereichen gekennzeichnet ist:
d(A) : 2,8 bis 3,0, 3,8 bis 4,1, 4,0 bis 4,5, 10 bis 12.

3. Verfahren gemäß Anspruch 2, gekennzeichnet dadurch, daß als zweiwertige Metalle Zink, Mangan, Magnesium, Eisen, Kobalt verwendet werden.

4. Verfahren gemäß Anspruch 1 bis 3, gekennzeichnet dadurch, daß als Bindemittel Pseudoböhmit, Kieselsäure oder Schichtsilikate, insbesondere Magadiit, verwendet werden.

5. Verfahren gemäß Anspruch 1 bis 4, gekennzeichnet dadurch, daß der Isomerisierungskatalysator ggf. eine hydrieraktive Komponente, insbesondere Palladium, in Anteilen von 0,01 bis 0,2 Masse-% enthält.

6. Isomerisierungskatalysatoren aus mikroporösem Alumophosphat mit Molekularsiebstruktur und Poreneingangsöffnungen von 0,4 bis 0,6 nm Durchmesser, zu deren Herstellung aktiviertes Siliziumdioxid eingesetzt wird, das vorwiegend aus sphärischen Partikeln mit einem Durchmesser von etwa 2 nm besteht und das ein IR-Spektrum gemäß Abb. 1 besitzt und dieses Alumophosphat mit einem Bindemittel gemischt und in bekannter Weise verformt und anschließend bei Temperaturen von 623 bis 873 K aktiviert wird.

7. Isomerisierungskatalysatoren nach Anspruch 6, gekennzeichnet dadurch, daß die Alumophosphate Silizium und/oder zweiwertige Metalle im Gerüst und/oder Gitter enthalten und ein Röntgendiffraktogramm (Cu-K-alpha-Strahlung), das durch Interferenzen in mindestens folgenden Netzebenenbereichen gekennzeichnet ist:
d(A) : 2,8 bis 3,0, 3,8 bis 4,1, 4,0 bis 4,5, 10 bis 12.

8. Isomerisierungskatalysatoren gemäß Anspruch 7, gekennzeichnet dadurch, daß als zweiwertige Metalle Zink, Mangan, Magnesium, Eisen und/oder Kobalt enthalten sind.

9. Isomerisierungskatalysatoren nach einem der Ansprüche 6 bis 8, gekennzeichnet dadurch, daß als Bindemittel Pseudoböhmit, Kieselsäure oder Schichtsilikate, insbesondere Magadiit, enthalten sind.

10. Isomerisierungskatalysatoren nach mindestens einem der Ansprüche 6 bis 9, gekennzeichnet dadurch, daß sie eine hydrieraktive Komponente, insbesondere Palladium, vorzugsweise in Anteilen von 0,01 bis 0,2 Masse-% enthalten.

## Claims

1. A process for the skeletal isomerization of n-alkenes to form isoalkenes and for the enrichment of isoalkenes in alkene-containing hydrocarbon mixtures, resp., at temperatures of 593 to 823 K and pressures of 98 to 2100 kPa and, if necessary, in presence of inert gases and/or hydrogen, on a microporous aluminophosphate catalyst with molecular sieve structure, **in which** a microporous aluminophosphate having pore inlet openings with a diameter of 0.4 to 0.6 nm is used as an isomerization catalyst, and for the production of which activated silicon dioxide with an IR spectrum as shown in Fig. 1 is used, and in which the said aluminophosphate is mixed with a binding agent and formed in known manner and then activated at temperatures of 623 to 873 K.

2. The process as claimed in claim 1 **in which** aluminophosphates are used which contain silicon and/or divalent metals in the skeleton and/or lattice and show a X-ray diffractogram (Cu-K-alpha radiation) which is characterized by interferences in, at least, the following lattice plane ranges:
d(A): 2.8 to 3.0; 3.8 to 4.1; 4.0 to 4.5; 10 to 12.

3. The process as claimed in claim 2 **in which** divalent metals such as zinc, manganese, magnesium, iron, cobalt are used.

4. The process as claimed in the claims 1 to 3 **in which** pseudoboehmite, silicic acid or sheet silicates, in particular, magadiite are used as binding agents.

5. The process as claimed in the claims 1 to 4 **in which** the isomerization catalyst may or may not contain a hydrogenation-active component, in particular, palladium, in portions of 0.01 to 0.2 % by weight.

6. Isomerization catalysts made of microporous aluminophosphate having a molecular sieve structure and pore inlet openings with a diameter of 0.4 to 0.6 nm, for the production of which activated silicon dioxide is used, which consists mainly of spherical particles with a diameter of about 2 nm and has an IR spectrum as shown in Fig. 1, and in which the said aluminophosphate is mixed with a binding agent and formed in known manner and then activated at temperatures of 623 to 873 K.

7. Isomerization catalysts as claimed in claim 6 **in which** the aluminophosphates contain silicon and/or divalent metals in the skeleton and/or lattice and show a X-ray diffractogram (Cu-K-alpha radiation) which is characterized by interferences in, at least, the following lattice plane ranges:
a(A): 2.8 to 3.0; 3.8 to 4.1; 4.0 to 4.5; 10 to 12.

8. Isomerization catalysts as claimed in claim 7 **in which** the said isomerization catalysts contain divalent metals such as zinc, manganese, magnesium, iron and/or cobalt.

9. Isomerization catalysts as claimed in any one of the claims 6 to 8 **in which** the said isomerization catalysts contain pseudoboehmite, silicic acid or sheet silicates, in particular, magadiite as binding agents.

10. Isomerization catalysts as claimed in, at least, any one of the claims 6 to 9 **in which** the said isomerization catalysts contain a hydrogenation-active component, in particular, palladium, preferably in portions of 0.01 to 0.2 % by weight.

## Revendications

1. procédé d'isomérisation de squelettes d'alcènes normales en isoalcènes et d'enrichissement d'isoalcènes en mélanges d'hydrocarbures à teneur d'alcènes, par des températures de 593 à 823 K et des pressions de 98 à 2 100 kPa, le cas échéant, en présence de gaz inertes et / ou d'hydrogène, avec un catalyseur à phosphate d'aluminium microporeux de structure de tamis moléculaire, caractérisé par le fait que le catalyseur d'isomérisation utilisé est un phosphate d'aluminium microporeux aux ouvertures d'entrée des pores ayant un diamètre de 0,4 à 0,6 nm, produit par l'emploi d'un dioxyde de silicium activé qui possède un spectre d'infrarouge conforme à la fig. 1, et que ce phosphate d'aluminium est mélangé à un liant et formé comme il faut, puis activé par des températures de 623 à 873 K.

2. Procédé d'après prétention 1, caractérisé par l'emploi de phosphates d'aluminium, contenant du silicium et / ou des métaux bivalents dans l'ossature ou dans le réseau et par un diagramme de diffraction des rayons X (rayonnement Cu-K-alpha), caractérisé par des interférences au moins dans les zones suivantes de niveaux de réseau:
d(A) : de 2,8 à 3,0, de 3,8 à 4,1, de 4,0 à 4,5 et de 10 à 12.

3. Procédé d'après prétention 2, caractérisé par le fait que sont utilisés comme métaux bivalents le zink, le manganèse, le magnésium, le fer, le cobalt.

4. Procédé selon les prétentions de 1 à 3, caractérisé par le fait que le liant utilisé est le pseudobohêmide, l'acide silicique ou des plyllosilicates, en particulier le magadiide.

5. Procédé selon les prétentions de 1 à 4, caractérisé par le fait que le catalyseur d'isomérisation contient éventuellement un composant hydrogémant actif, comme en particulier le palladium, en parts de 0,01 à 0,2 % en masse.

6. Catalyseurs d'isomérisation à phosphate d'aluminium microporeux de structure de tamis moléculaire, et ouvertures d'entrée des pores ayant a diamètre de 0,4 à 0,6 nm, produits par l'emploi d'un dioxyde de silicium activé constitué principalement de particules sphériques au diamètre d'environ 2 nm et qui possèdent un spectre d'infrarouge conforme à la fig. 1, et que ce phosphate d'aluminium est mélangé à un liant et formé comme il faut, puis activé par des températures de 623 à 873 K.

7. Catalyseurs d'isomérisation selon la prétention 6, caractérisés par l'emploi de phosphates d'aluminium, contenant du silicium et / ou des métaux bivalents dans la structure ou dans le réseau et par un diagramme de diffraction des rayons X (rayonnement Cu-K-alpha), caractérisé par des interférences au moins dans les zones suivantes de niveaux de réseaux:
a(A) : de 2,8 à 3,0, de 3,8 à 4,1, de 4,0 à 4,5 et de 10 à 12.

8. Catalyseurs d'isomérisation selon la prétention 7, caractérisés par le fait que sont contenus comme métaux bivalents le zink, le manganèse, le magnésium, le fer, le cobalt.

9. Catalyseurs d'isomérisation selon une des prétentions de 6 à 8, caractérisés par le fait que le liant contenu est du pseudobohêmide, de l'acide silicique ou des phyllosilicates, en particulier du magadiide.

10. Catalyseurs d'isomérisation selon au moins une des prétentions de 6 à 9, caractérisés par le fait qu'ils contiennent éventuellement un composant hydrogénant actif, comme en particulier le palladium, en parts de 0,01 à 0,2 % en masse.
